# EUROPEAN PATENT SPECIFICATION

(11) **EP 4 472 945 B1**
(45) Date of publication and mention of the grant of the patent: **27.05.2026**
(21) Application number: 23701481.6
(22) Date of filing: 23.01.2023
(51) Int. Cl.: C07C 329/16, C08F 8/06, C08F 216/14, C08F 214/26, C08F 228/02

(54) **METHOD FOR PREPARING FUNCTIONALIZED POLYMERS**
VERFAHREN ZUR HERSTELLUNG VON FUNKTIONALISIERTEN POLYMEREN
PROCÉDÉ DE PRÉPARATION DE POLYMÈRES FONCTIONNALISÉS

(30) Priority: 04.02.2022 EP 22155260
(43) Date of publication of application: 11.12.2024
(73) Proprietor: Syensqo Specialty Polymers Italy S.p.A., 20021 Bollate (MI) (IT)
(72) Inventor: OLDANI, Claudio, 20015 Parabiago (MI) (IT); MONZANI, Cristiano, 20056 Trezzo sull'Adda (MI) (IT)
(74) Representative: Ferri, Isabella
(86) International application number: PCT/EP2023/051463
(87) International publication number: WO 2023/148027

(56) References cited:
- WO-A1-2014/173782
- CN-A- 103 304 945
- CN-A- 106 046 231
- CN-A- 113 999 337
- US-A- 2 853 506

## Description

### Reference to related applications

This application claims priority from European patent application Nr. 22155260.7 filed on 4 February 2022.

### Technical Field

The present invention provides compounds that include a fluorinated allyl xanthate group. The invention also relates to a process for making these compounds and the use of said compounds as chain transfer agents or as monomers. The invention relates also to polymers, including copolymers, comprising recurring units deriving from compounds comprising fluorinated allyl xanthate groups.

### Background Art

In certain instances, it is beneficial to provide polymers comprising functional groups.

Polymers with attached functional groups may be prepared directly by polymerization of functional monomers. Oligomers and polymers prepared by a controlled polymerization processes may have functionality at specific locations along the chain and a specific amount of functionality. For example, functional monomers may be placed periodically along the polymer chain, the initiator may have attached functionality, or the group providing for controlled polymerization may be removed and replaced with a desired functional group. However, there are several functional monomers that may not be directly copolymerized by polymerization process. Further, the monomers with desired functionality may not copolymerize in the desired manner using the selected polymerization process.

For instance, introducing monomers bearing -CF₂SO₃H and -CF₂SH side chain functional groups is very difficult.

The access to polymers having super short side chains bearing -CF₂SO₃H groups in the field of fuel cell and electrolysis applications would lead to systems, such as membranes, higher electrochemical performance and better mechanical properties than the current technology.

The Applicant has found a novel monomer, more specifically a monomer comprising a fluorinated allyl xanthate, which can be used for the synthesis of polymers bearing pendant functionalities that can suitably be converted into several functional groups in post-polymerization processes. The resulting polymers can be tailored to different applications.

Alkyl xanthates are compounds having the general formula ROC(=S)SR'. They are widely used in the field of engineering such as flotation and as chain transfer agents in controlled radical polymerizations, and they are generally prepared by substitution reaction of xanthates with chloro alkyl compounds. CN 106 046 231 A and CN 113 999 337 A disclose co-polymers comprising repeating moieties formally derived from CF₂=CF-CF₂-SO₃Na.

### Summary of invention

Thus a first object of the present invention is a compound (AX) complying with formula (I): 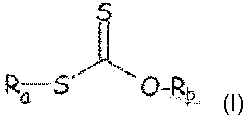 wherein Rₐ is a (per)fluorinated allyl group and R_{b} is a straight or branched alkyl group. R_{b} is a C₁-C₁₂ straight or branched alkyl group, typically a C₁-C₈ straight or branched alkyl group, preferably a C₁-C₆ straight or branched alkyl group.

The invention also relates to processes for preparing the compound (AX) according to the first object.

Another object of the present invention is a polymer (P) comprising recurring units deriving from compound (AX) of formula (I) as above defined.

The invention also relates to a process for manufacturing a polymer (P) comprising recurring units deriving from compound (AX) of formula (I) as above defined.

Polymer (P) may suitably be subjected to chemical transformations in order to convert the -S(=S)OR_{b} groups present in the recurring units deriving from compound (AX) into different functional groups, thus providing further functionalized polymers.

In a further object, the present invention is directed to the use of a compound (AX) of formula (I) as above defined as chain transfer agent in controlled radical polymerizations.

### Description of embodiments

In the present application:
- any description, even though described in relation to a specific embodiment, is applicable to and interchangeable with other embodiments of the present disclosure;
- where an element or component is said to be included in and/or selected from a list of recited elements or components, it should be understood that in related embodiments explicitly contemplated here, the element or component can also be any one of the individual recited elements or components, or can also be selected from a group consisting of any two or more of the explicitly listed elements or components; any element or component recited in a list of elements or components may be omitted from such list;
- any recitation herein of numerical ranges by endpoints includes all numbers subsumed within the recited ranges as well as the endpoints of the range and equivalents;
- the use of parentheses "(...)" before and after symbols or numbers identifying formulae or parts of formulae has the mere purpose of better distinguishing that symbol or number with respect to the rest of the text; thus, said parentheses could also be omitted.

For the purpose of the invention, the term "(per)fluorinated allyl group" is intended to denote any partially or fully fluorinated allyl group, i.e. wherein all or only a part of the hydrogen atoms of the hydrocarbon allyl structure have been replaced by fluorine atoms attached to unsaturated and/or to saturated carbons. When the allyl group is fully fluorinated, the term perfluorinated is used.

Rₐ is preferably a perfluorinated allyl group.

The compound (AX) of the present invention preferably complies with formula (II) wherein R_{b} is as defined above:

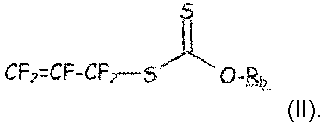

Non-limitative examples of R_{b} include, notably: ethyl, isopropyl, n-butyl, isobutyl, n-pentyl and isopentyl groups.

Particularly preferred is a compound (AX) of formula (III): 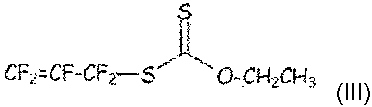 , hereinafter referred to as "FAX".

Compound (AX) of the present invention can be prepared by a process comprising the following steps a) and b):
a) providing a xanthate salt of formula (IV): 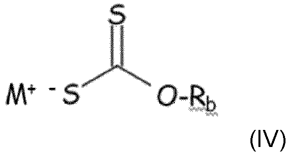 wherein R_{b} is as above defined and M⁺ is a monovalent cation;
b) reacting the xanthate salt provided in step a) with a (per)fluoro allyl fluorosulfate of formula (V):

   Rₐ-OSO₂X (V)

   wherein Rₐ is a (per)fluorinated allyl group and X is a halogen atom.

In formula (IV) M⁺ is preferably selected from alkali metal cations, more preferably M⁺ is selected from Na⁺, K⁺, Cs⁺ and Li⁺, even more preferably M⁺ is K⁺.

Among the (per)fluoro allyl fluorosulfates, perfluoro allyl fluorosulfate of formula CF₂=CFCF₂OSO₂F (hereinafter referred to as "FAFS") is particularly preferred.

In step b), the reaction is carried out preferably at room temperature.

The reaction in step b) is typically carried out in the presence of a solvent. Suitable solvents for the reaction in step b) are polar aprotic solvents, notably glycol ethers, ethers, nitriles. Preferably the solvent is acetonitrile.

The reaction time in step b) is suitably comprised between 1 and 5 hours.

At the end of step b), the solid FSO₃M by-product is filtered off from the reaction mixture and compound (AX) is recovered in the form of powder after evaporation of the solvent.

Compound (AX) of the present invention can be used in the preparation of polymers. Advantageously, the recurring units deriving from compound (AX) can serve as a precursor to other protective and/or reactive functionalities such as -CF₂SO₃H and -CF₂SH.

Hence a further object of the invention is a polymer (P) comprising recurring units deriving from compound (AX).

Polymer (P) may be a homopolymer. That is, polymer (P) may consist of recurring units deriving from compound (AX).

Alternatively, polymer (P) may be a copolymer, comprising recurring units deriving from compound (AX) and recurring units deriving from one or more ethylenically unsaturated monomers.

Polymer (P) of the present invention is preferably a copolymer.

More preferably, polymer (P) is a copolymer comprising recurring units deriving from compound (AX) as above defined and recurring units deriving from at least one fluoromonomer [fluoromonomer (FM)]. The expression "fluoromonomer" is used herein according to its usual meaning, that is to say for designating an ethylenically unsaturated monomer comprising at least one fluorine atom.

In a preferred embodiment of the invention, polymer (P) comprises 85 to 5 % by moles of recurring units deriving from compound (AX), with respect to the total moles of recurring units of polymer (P), and 15 to 95 % by moles of recurring units deriving from the at least one fluoromonomer (FM), with respect to the total moles of recurring units of polymer (P).

Polymer (P) may comprise at least 5 %, at least 10 %, at least 15%, at least 20% at least 25 %, at least 35 %, at least 45%, at least 50 %, even at least 60 %, at least 70 % by moles of compound (AX) with respect to the total moles of recurring units of polymer (P). Polymer (P) may comprise less than 80 %, less than 75%, less than 65% and even less than 50%, less than 45%, less than 30 % by moles of compound (AX) with respect to the total moles of recurring units of polymer (P). The remainder of recurring units in polymer (P) derives from one or more fluoromonomer (FM).

Fluoromonomer (FM) is selected generally from the group consisting of:
- C₂-C₈ perfluoroolefins, such as tetrafluoroethylene, hexafluoropropene;
- C₂-C₈ hydrogen-containing fluoroolefins, such as vinyl fluoride, 1,2-difluoroethylene, vinylidene fluoride, trifluoroethylene, pentafluoropropylene, and hexafluoroisobutylene;
- (per)fluoroalkylethylenes complying with formula CH₂=CH-R_{f0}, in which R_{f0} is a C₁-C₈ (per)fluoroalkyl or a C₁-C₈ (per)fluorooxyalkyl having one or more ether groups ;
- chloro- and/or bromo- and/or iodo-C₂-C₆ fluoroolefins, like chlorotrifluoroethylene;
- fluoroalkylvinylethers complying with formula CF₂=CFOR_{f1} in which R_{f1} is a C₁-C₆ fluoro- or perfluoroalkyl, e.g. -CF₃, -C₂F₅, -C₃F₇ ;
- hydrofluoroalkylvinylethers complying with formula CH₂=CFOR_{f1} in which R_{f1} is a C₁-C₈ fluoro- or perfluoroalkyl, e.g. -CF₃, -C₂F₅, -C₃F₇ ;
- fluoro-oxyalkylvinylethers complying with formula CF₂=CFOX₀, in which X₀ is a C₁-C₁₂ oxyalkyl, or a C₁-C₁₂ (per)fluorooxyalkyl having one or more ether groups, like perfluoro-2-propoxy-propyl;
- fluoroalkyl-methoxy-vinylethers complying with formula CF₂=CFOCF₂OR_{f2} in which R_{f2} is a C₁-C₈ fluoro- or perfluoroalkyl, e.g. - CF₃, -C₂F₅, -C₃F₇ or a C₁-C₆ (per)fluorooxyalkyl having one or more ether groups, like -C₂F₅-O-CF₃;
- functional fluoro-alkylvinylethers complying with formula CF₂=CFOY₀, in which Y₀ is a C₁-C₁₂ alkyl or (per)fluoroalkyl, or a C₁-C₁₂ oxyalkyl or a C₁-C₁₂ (per)fluorooxyalkyl, said Y₀ group comprising a carboxylic or sulfonic acid group, in its acid, acid halide or salt form;
- fluorodioxoles, of formula: 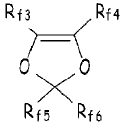 wherein each of R_{f3}, R_{f4}, R_{f5}, R_{f6}, equal or different each other, is independently a fluorine atom, a C₁-C₈ fluoro- or per(halo)fluoroalkyl, optionally comprising one or more oxygen atom, e.g. -CF₃, -C₂F₅, -C₃F₇, - OCF₃, -OCF₂CF₂OCF₃.

Polymer (P) may comprise recurring units deriving from at least one additional monomer different from fluoromonomer (FM), that is to say a monomer free from fluorine, otherwise generally referred to as a hydrogenated monomer [monomer (HM)]. Examples of hydrogenated monomers (HM) are notably C₂-C₈ non-fluorinated olefins, in particular C₂-C₈ non-fluorinated alpha-olefins, including ethylene, propylene, 1-butene; diene monomers; styrene monomers. Monomer (HM) is preferably selected from with C₂-C₈ alpha-olefins.

In one preferred embodiment of the present invention, polymer (P) is a copolymer comprising recurring units deriving from compound (AX) as above defined, recurring units deriving from at least one C₂-C₈ perfluoroolefin and recurring units deriving from at least one functional fluoro-alkylvinylether of formula CF₂=CFOY₀ as above defined.

The C₂-C₈ perfluoroolefin is preferably tetrafluoroethylene. The functional fluoro-alkylvinylether of formula CF₂=CFOY₀ is preferably selected from the fluoro-alkylvinylethers in which Y₀ is a C₁-C₁₂ (per)fluorooxyalkyl group comprising a sulfonic acid group, in its acid, acid halide or salt form.

The functional fluoro-alkylvinylether of formula CF₂=CFOY₀ is preferably selected from the group consisting of:
(j) sulfonyl halide fluorovinylethers of formula: CF₂=CF-O-(CF₂)_{m'}SO₂X', wherein m' is an integer between 1 and 10, preferably between 1 and 6, more preferably between 2 and 4, even more preferably m' equals 2; and X' is chosen among halogens (Cl, F, Br, I), preferably F, or is -O⁻M'⁺, wherein M'⁺ is a cation selected among H⁺, NH₄⁺, K⁺, Li⁺, Na⁺, or mixtures thereof, preferably M'⁺ is H⁺; and
(jj) sulfonyl fluoride fluoroalkoxyvinylethers of formula:

   CF₂=CF-(OCF₂CF(R_{F1}))_{w}-O-CF₂(CF(R_{F2}))_{y}SO₂X'

   wherein X' is chosen among halogens (Cl, F, Br, I), preferably F, or is -O⁻M'⁺, wherein M'⁺ is a cation selected among H⁺, NH₄⁺, K⁺, Li⁺, Na⁺, or mixtures thereof, preferably M'⁺ is H⁺; and wherein w is an integer between 0 and 2, R_{F1} and R_{F2}, equal or different from each other, are independently F, Cl or a C₁-C₁₀ fluoroalkyl group, optionally substituted with one or more ether oxygens, y is an integer between 0 and 6; preferably w is 1, R_{F1} is - CF₃, y is 1 and R_{F2} is F.

Still more preferably, the at least one functional fluoro-alkylvinylether is a sulfonated perfluorovinylether of formula (FM1): 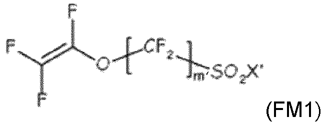 wherein m' is an integer between 1 and 10, preferably between 1 and 6, more preferably between 2 and 4, and X' is chosen among halogens (Cl, F, Br, I), preferably F, -O⁻M'⁺, wherein M'⁺ is a cation selected among H⁺, NH₄⁺, K⁺, Li⁺, Na⁺, or mixtures thereof, preferably M'⁺ is H⁺.

Advantageously, the sulfonated perfluorovinylether of formula (FM1) is selected in the group consisting of the compounds of formulae (FM1-A), (FM1-B) and (FM1-C): 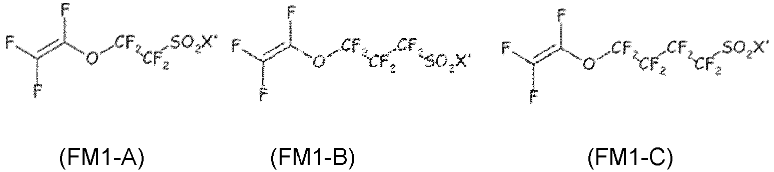 wherein X' has the same meaning as above defined.

The sulfonated perfluorovinylether is preferably perfuoro-5-sulfonylfluoride-3-oxa-1-pentene (hereinafter referred to as " VEFS") of formula (FM1-D): 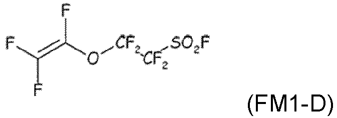 which can be in its -SO₂F form as shown above or it can be in its -SO₃X form, with X being H or alkaline metal or NH⁴⁺.

Advantageously, polymer (P) is a copolymer of formula (VI): 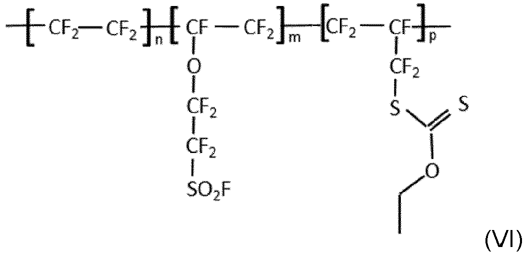 wherein n, m and p are, independently from each other, integers greater than 0, together denoting the molar fraction of each monomer in the polymer (P). Typically n is between 0.50 and 0.75, m is between 0.10 and 0.30 and p is between 0.05 and 0.40.

In the above notation for copolymers, the n, m and p units may appear in any order: the formula (VI) is only intended to define the relative proportion of monomer units, and not the exact order (which is random) in the copolymer. Similarly, the orientation of the recurring units in a tail-to-tail pattern in formula (VI) is only indicative and not intended to limit the structure of the polymer. The recurring units in the polymer (P) of formula (VI) may be in a head-to-head, tail-to-tail or head-to-tail arrangement.

In a preferred embodiment of the invention, polymer (P) comprises:
- 5 to 25 %, 10 to 20 % by moles of recurring units deriving from compound (AX),
- 45 to 85 %, 60 to 70 % by moles of recurring units deriving from tetrafluoroethylene, and
- 10 to 30 %, 15 to 25% by moles of recurring units deriving from the functional fluoro-alkylvinylether of formula (FM1-D),
the amount by moles being referred to the total moles of recurring units of polymer (P).

Polymer (P) may be prepared by a process that comprises the polymerization of a monomer mixture (MM) comprising:
(i) at least one compound (AX) as above defined;
(ii) at least one fluoromonomer (FM) as above defined;
in the presence of at least free radical initiator and optionally at least one surfactant. The polymerization may be performed in an aqueous emulsion.

The monomer mixture (MM) may optionally comprise: (iii) at least one monomer different from fluoromonomer (FM), that is to say a monomer (HM) as above defined.

Monomer mixtures (MM) comprising a compound (AX), one or more than one fluoromonomer (FM) and optionally monomer (HM) are generally employed in the preparation of polymer (P) of the present invention.

The polymerization initiators used in the process of the present invention are organic or inorganic. As organic initiators, diisopropyl peroxydicarbonate (IPP) or di-tert-butyl peroxide (DTBP) can for example be mentioned. Radical inorganic initiators, such for example ammonium and/or potassium and/or sodium persulfate, optionally in combination with ferrous, cupreous or silver salts, are preferably used. The initiator feeding procedures can be in a continuous way or by a single addition at the start of the polymerization.

A surfactant may optionally be used. The surfactant may be a fluorinated or a non-fluorinated surfactant.

Among fluorinated surfactants mention may be made of functional (per)fluoropolyether compounds comprising at least one (per)fluoropolyoxyalkylene chain and at least one functional end-group selected from carboxylic acid, phosphonic acid and sulfonic acid groups as well as cyclic fluorocompounds such as those described in WO 2010/00392.

In a typical polymerization process, formation of a mixture containing water, an optional surfactant and the monomers (i), (ii) and optionally (iii), at the polymerization temperature, is placed in a reaction vessel; the polymerization reaction is started by addition of the free radical initiator. In an emulsion polymerization process a surfactant is generally present in the mixture, thereby forming an emulsion.

The polymerization reaction is generally carried out at temperatures in the range 25°-130° C. The polymerization is typically performed at atmospheric pressure or under pressure, for example from 2 bar up to 60 bar.

Preferably the polymerization reaction is generally carried out at temperatures in the range 40°-70° C, preferably 50°-60° C, under pressure up to 20 bar, preferably higher than 5 bars.

A polymerization latex or suspension comprising the polymer dispersed in an aqueous liquid phase is obtained at the end of the process. The polymer (P) can be recovered from said polymerization latex using well-known techniques, such as a freeze-thawing coagulation method or by or addition of electrolytes such as aluminum sulfate or nitric acid.

Additional treatments such as purification of the latex or suspension, washing from contaminants, drying and the like can be performed on such coagulum before isolating polymer (P) in the form of powder.

Advantageously, a polymer (P) of the present invention is preferably obtained by a process comprising the polymerization of a monomer mixture (MM) comprising a compound (AX) of formula (III) as above defined, tetrafluoroethylene and a fluoromonomer (FM) of formula (FM1-D). A polymer (P) of formula (VI) as defined above is thus obtained.

Polymer (P) according to the present invention may be subjected to a chemical transformation.

Advantageously, the -S(=S)OR_{b} groups present in the recurring units deriving from compound (AX) may be converted into different functional groups.

In a further aspect, the present invention thus provides a process for the chemical transformation of recurring units deriving from compound (AX) of polymer (P) into different functional groups.

According to a first embodiment of the present invention, the process comprises the chemical hydrolysis of the xanthate moiety of the recurring units deriving from compound (AX), namely the moiety of formula 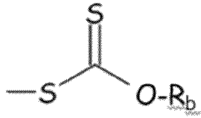 , to the group -SH.

Chemical hydrolysis of the xanthate moiety to sulphydryl group -SH may be suitably carried out in the presence of acid or basic aqueous solutions. Hence, the process comprises reacting polymer (P) with an acid or a base in an aqueous solution.

Suitable acid aqueous solutions are notably solutions comprising HCl, HBr or H₃PO₄.

Suitable basic aqueous solutions are notably solutions comprising NaOH or KOH.

The reaction is generally carried out at a temperature in the range 25°-100° C.

Conversion of the -S(=S)OR_{b} groups into -SH groups may be observed by analytical techniques such as infrared spectroscopy.

According to a further embodiment of the present invention, the process of chemical transformation to be applied on polymer (P) is represented by conversion of the xanthate moiety of the recurring units deriving from compound (AX), namely the moiety of formula 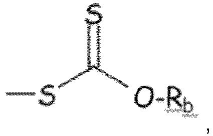 to the group -SO₃H by chemical oxidation.

Chemical oxidation of the xanthate moiety to sulfonic acid group -SO₃H may be suitably carried out in the presence of an oxidant such as hydrogen peroxide. Hence, the process comprises the reaction of polymer (P) with an oxidant, preferably hydrogen peroxide.

The reaction is generally carried out at temperatures in the range 25°-100° C.

Conversion of the -S(=S)OR_{b} groups into -SO₃H groups may be observed by analytical techniques such as infrared spectroscopy.

Hence a further object of the invention is a polymer (P^{ox}) of formula (VII): 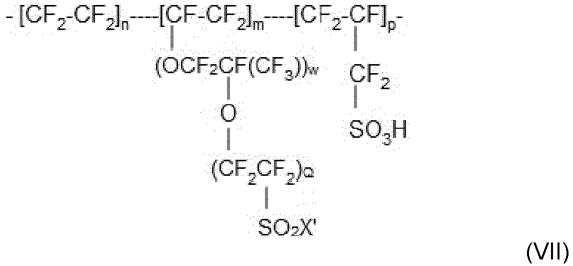 wherein: w is either 0 or 1, Q is an integer between 1 and 4, preferably 1 or 2; X' is selected among halogens (Cl, F, Br, I), preferably F or is -O⁻M'⁺, wherein M'⁺ is a cation selected among H⁺, NH₄⁺, K⁺, Li⁺, Na⁺, or mixtures thereof, preferably M'⁺ is H⁺; n and p are, independently from each other, integers greater than 0; m is equal to greater than 0; m, n and p denoting the molar fraction of each monomer in the polymer. Typically, n is between 0.50 and 0.75, m is between 0 and 0.30 and p is between 0.05 and 0.50. In formula (VII), n may be between 0.60 and 0.70, m between 0.10 and 0.30 and p between 0.10 and 0.20.

In a particularly preferred embodiment of the present invention, a polymer (P) of formula (VI) as above defined is post treated by chemical oxidation with hydrogen peroxide to provide a post treated polymer (P^{ox}) of formula (VIII): 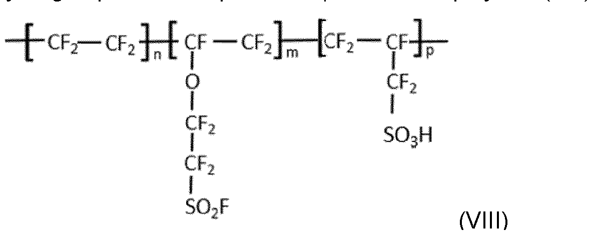 wherein n, m and p are independently from each other integers greater than 0, denoting the molar fraction of each monomer in the polymer. Typically n is between 0.50 and 0.65, m is between 0.10 and 0.30 and p is between 0.05 and 0.40. In formula (VIII) n may be between 0.60 and 0.70, m between 0.10 and 0.30 and p between 0.10 and 0.20.

In the above notation for copolymers, the n, m and p units may appear in any order: formula (VII) and (VIII) are only intended to define the relative proportion of monomer units, and not the exact order (which is random) in the copolymer. Similarly, the orientation of the recurring units in a tail-to-tail pattern in formulae (VII) and (VIII) is only indicative and not intended to limit the structure of the polymer. The recurring units in the polymer of formula (VII) or (VIII) may be in a head-to-head, tail-to-tail or head-to-tail arrangement.

Polymers (P^{ox}) such as those of formula (VII) or (VIII) obtained from the inventive process are ion conducting polymers or precursors thereof. They are particularly suitable to be used in electrochemical applications. Polymers of formula (VII) or (VIII) may be used in the preparation of membranes for fuel cells, of membranes for electrochemical applications, such for example chloro-soda cells, lithium batteries. They may additionally be used as membranes in electrodialysis applications and in reactors in which membranes made of the polymer act as a superacid catalyst.

Sulfonic perfluorinated ion conducting polymers, owing to the combination of chemical stability in harsh environments and good proton conductivity in a wide range of humidity conditions, are today considered the material benchmark for fuel cells (mainly low temperature fuel cells for transportation) and electrolyzers (producing the so-called green hydrogen from renewables). Commercially available perfluorinated ion conducting polymers are copolymers of tetrafluoroethylene and vinyl ethers of different length bearing -SO₃H groups. The ion conducting polymer having the shortest side chain (two -CF₂- groups) currently available on the market is Aquivion^{®} ion conducting polymer from Solvay whereas the ion conducting polymer with the longest side chain is Nafion^{®} from Chemours. The access to ion conducting polymer with shorter side chains would have great impact in increasing electrochemical performance and mechanical strength.

This invention thus gives access to perfluorinated ion conducting polymer having the side chain containing only one -CF₂- group. Polymers (P) in which the -S(=S)OR_{b} group has been oxidized to the -SO₃H group, including polymers of formula (VII) or (VIII), are advantageously endowed with higher proton conductivity, higher crystallinity and higher mechanical strength than perfluorinated ion conducting polymers available on the market.

A further object of the invention is therefore an article comprising polymer (P) or polymer (P^{ox}). The article could be in the form of a membrane, such as an ion conducting membrane. The ion conducting membrane comprising polymer (P) or, advantageously, polymer (P^{ox}) might be used in electrolysis applications or in fuel cell applications.

In another aspect of the present invention, the use of a compound (AX) of formula (I) as above defined as chain transfer agent in controlled radical polymerizations is provided.

Among controlled radical polymerization techniques, reversible addition-fragmentation chain transfer (RAFT) and macromolecular design via interexchange of xanthate compounds (MADIX) can be mentioned.

RAFT/MADIX agents are capable to act as a reversible chain transfer agent in free-radical polymerizations, thereby inducing reversible-addition fragmentation transfer reactions to create an equilibrium between propagating radicals (i.e. the growing polymer chain) and so-called dormant species (containing the chain transfer agent fragment) that can become active again.

The Applicant has surprisingly found that compound (AX) can be suitably used as RAFT/MADIX agent in emulsion polymerization of fluorinated monomers in order to control microstructure of the polymer.

Thus, in a further aspect, the present invention provides a method for emulsion polymerization of at least one fluoromonomer, said method comprising:
(i) providing at least one aqueous emulsion comprising a monomer mixture comprising at least one fluoromonomer [monomer (F)] and at least one compound (AX) of formula (I): 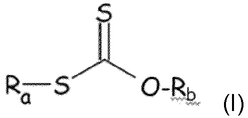 wherein Rₐ is a (per)fluorinated allyl radical group, R_{b} is a straight or branched alkyl radical group and optionally at least one surfactant;
(ii) initiating the polymerization of said monomer mixture in said aqueous emulsion adding at least one radical initiator;
(iii) continuing the polymerization by adding additional amounts of said at least one monomer (F) and/or said compound (AX), until converting the targeted amount of said monomer mixture, and
(iv) terminating the polymerization and recovering a latex of fluoropolymer [polymer (F)].

The expression "fluoromonomer" is used herein according to its usual meaning, that is to say for designating an ethylenically unsaturated monomer comprising at least one fluorine atom.

Fluoromonomer (F) can notably be a fluoromonomer (FM) as above defined.

The method of the invention is suitable for the manufacture of a large variety of fluoropolymers, including notably non-melt processable tetrafluoroethylene polymers (including PTFE homopolymers and its copolymers comprising low amounts of perfluorinated comonomers), thermoplastic fluoropolymers (e.g. vinylidene fluoride homopolymers and its plastomeric copolymers, copolymers of ethylene with chlorotrifluoroethylene, thermoplastic copolymers of tetrafluoroethylene and perfluoroalkyl vinylethers, thermoplastic copolymers of tetrafluoroethylene and hexafluoropropylene), and fluoroelastomers.

The invention will be now described with reference to the following examples, whose purpose is merely illustrative and not intended to limit the scope of the invention.

### Experimental section

### EXAMPLE 1: FAX synthesis

A three-necked round bottom flask equipped with a thermometer, a condenser and a dropping funnel was charged with 109.20 g of CF₂=CFCF₂OSO₂F (FAFS) under nitrogen. Then, 70.57 g of Potassium Ethylxanthogenate dissolved in 1482 ml of Acetonitrile was added dropwise in 25 minutes, at room temperature. After two hours of stirring the reaction was complete and a white solid (FSO₃K) precipitated. The white solid was filtered and the resulting clear solution was washed three times with distilled water (1:1 volume vs organic phase). The organic phase was separated and distilled under vacuum obtaining 82.73 g of pure CF₂=CFCF₂S(C=S)OCH₂CH₃ (FAX).

¹⁹F NMR in Acetone (HFMX reference): -82 ppm (m; 2F; -SCF₂CF=CF₂); - 93.5 ppm (m; 1F; cis -SCF₂CF=CF₂); -105 ppm (m; 1F; trans - SCF₂CF=CF₂); -184 ppm (m; 1F; -SCF₂CF=CF₂). ¹H NMR in Acetone (TMS reference): +4.8 ppm (q; 2H; -OCH₂CH₃); +1.5 ppm (m; 3H; - OCH₂CH₃).

### EXAMPLE 2: Polymerization of TFE+VEFS+FAX

Deionized water (1.8L), VEFS (212 g) and solution in water of Fluorolink 7800 (540 g, 5 wt%) were loaded in a 5L reactor and then it was pressurized with 7.5 bar of TFE and the system was heated to 50°C under stirring. The reaction took place after the feeding of a solution of potassium persulfate (conc. 10.5 g/L, 200 mL). Every 10% TFE conversion, 45 g of VEFS and 50 g of a solution of VEFS (90 wt%) and FAX (10 wt%) were fed. When 4.95 g of FAX and 257 g of VEFS were added, the reaction was stopped, cooled down and the pressure was reduced by removing TFE. The polymer latex thus obtained was freeze-thawed and the polymer was recovered as a yellowish powder.

FT-IR: 970 cm-1 (combined symmetric stretching of C-F and C-O-C); 1150 cm-1 (combined asymmetric stretching of C-O-C and stretching vibrations of C-F bonds); 1020 cm⁻¹ (C=S stretching); 1220 cm⁻¹ (symmetric and asymmetric stretching of CF₂); 1470 cm-1 (S-F bond motion); 2365 cm⁻¹ (C-F overtone/combination band); 2705 cm⁻¹ (S-F overtone).

### EXAMPLE 3 (comparative): Polymerization of TFE+VEFS

Deionized water (1.8 L), VEFS (212 g) and solution in water of Fluorolink 7800 (540 g, 5 wt%) were loaded in a 5L reactor and then it was pressurized with 7.5 bar of TFE and the system was heated to 50°C under stirring. The reaction took place after the feeding of a solution of potassium persulfate (conc. 10.5 g/L, 200 mL). Every 10% TFE conversion, 45 g of VEFS were fed. When 707 g of VEFS were added, the reaction was stopped, cooled down and the pressure was reduced by removing TFE. The polymer latex thus obtained was freeze-thawed and the polymer was recovered as a white powder. The powder was washed four times with demineralized water (1 L) at room temperature and under stirring and then dried in a vent oven at 80°C overnight.

FT-IR: 970 cm-1 (combined symmetric stretching of C-F and C-O-C); 1150 cm⁻¹ (combined asymmetric stretching of C-O-C and stretching vibrations of C-F bonds); 1220 cm⁻¹ (symmetric and asymmetric stretching of CF₂); 1470 cm⁻¹ (S-F bond motion); 2365 cm⁻¹ (C-F overtone/combination band); 2705 cm⁻¹ (S-F overtone).

### EXAMPLE 4: Transformation of the xanthate group into the -SO₃H group

The polymer from the EXAMPLE 2 was washed four times with demineralized water (1 L each) and with ethyl acetate (0.5 L). The washings were carried out under stirring and at room temperature. The powder was then dried in a vent oven at 80°C overnight. The polymer was stirred for 8 h at 45°C in a solution of H₂O₂ (15%, 200 mL) and H₂SO₄ (0.5 M, 2 mL) having pH of about 4. The powder thus obtained was washed with distilled water four times (1 L each) at room temperature and under stirring and finally dried in a vent oven at 80°C overnight.

FT-IR: 515 cm-1 (C-S deformation of CF₂-**SO**₃); 634 cm-1 (S-OH deformation of SO₃H); 970 cm-1 (combined symmetric stretching of C-F and C-O-C); 1057 cm-1 (symmetric stretching of SO₃); 1154 cm-1 (combined asymmetric stretching of C-O-C and SO₃ and stretching vibrations of C-F bonds); 1220 cm-1 (symmetric and asymmetric stretching of CF₂); 1300 cm-1 (symmetric and asymmetric stretching of SO₃); 1470 cm-1 (S-F bond motion); 2365 cm-1 (C-F overtone/combination band); 2705 cm-1 (S-F overtone).

### EXAMPLE 5 (comparative): Transformation of the -SO₂F group into the -SO₃H group

The polymer from the comparative EXAMPLE 3 was treated with a solution of NaOH in demineralized water (20 wt%, 1 L) at 80°C under stirring. After 8 h the powder was washed four times with demineralized water (1 L each) under stirring and at room temperature and then treated twice with a solution of HNO₃ in distilled water (20 wt%, 1 L each) at room temperature and under stirring. The polymer was washed under stirring with distilled water (4 x 1 L) at room temperature and then dried in a vent oven (80°C, overnight).

FT-IR: 515 cm⁻¹ (C-S deformation of CF₂-SO₃); 634 cm⁻¹ (S-OH deformation of SO₃H); 970 cm⁻¹ (combined symmetric stretching of C-F and C-O-C); 1057 cm⁻¹ (symmetric stretching of SO₃); 1154 cm⁻¹ (combined asymmetric stretching of C-O-C and SO₃ and stretching vibrations of C-F bonds); 1220 cm⁻¹ (symmetric and asymmetric stretching of CF₂); 1300 cm⁻¹ (symmetric and asymmetric stretching of SO₃).

## Claims

1. A compound (AX) of formula (I): 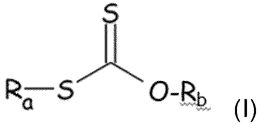 wherein Rₐ is a (per)fluorinated allyl group and R_{b} is a straight or branched alkyl group, preferably R_{b} is a C₁-C₁₂ straight or branched alkyl group.

2. The compound (AX) according to claim 1 complying with formula (II): 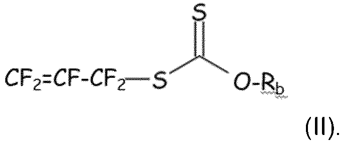 wherein R_{b} is selected from the group consisting of: ethyl, isopropyl, n-butyl, isobutyl, n-pentyl and isopentyl groups, preferably ethyl.

3. A process for the preparation of the compound (AX) according to claim 1, said process comprising the following steps a) and b):
a) providing a xanthate salt of formula (IV) 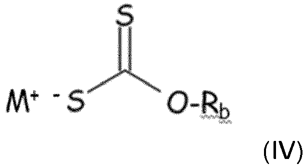 wherein R_{b} is a straight or branched alkyl group and M⁺ is a monovalent cation preferably selected from alkali metal cations, more preferably M⁺ is selected from Na⁺, K⁺, Cs⁺ and Li⁺, even more preferably M⁺ is K⁺,
b) reacting the xanthate salt provided in step a) with a (per)fluoro allyl fluorosulfate of formula (V)
Rₐ-OSO₂X (V)
wherein Rₐ is a (per)fluorinated allyl group and X is a halogen atom.

4. A polymer (P) comprising recurring units deriving from compound (AX) of formula (I) 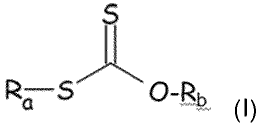 wherein Rₐ is a (per)fluorinated allyl group and R_{b} is a straight or branched alkyl group.

5. Polymer (P) according to claim 4, which is a copolymer comprising recurring units deriving from compound (AX), preferably in an amount of 85 to 5 % by moles with respect to the total moles of recurring units of polymer (P), and recurring units deriving from at least one ethylenically unsaturated monomer comprising at least one fluorine atom [fluoromonomer (FM)], preferably in an amount of 15 to 95 % by moles with respect to the total moles of recurring units of polymer (P).

6. Polymer (P) according to claim 5, wherein the fluoromonomer (FM) is selected from the group consisting of:
- C₂-C₈ perfluoroolefins, such as tetrafluoroethylene, hexafluoropropene;
- C₂-C₈ hydrogen-containing fluoroolefins, such as vinyl fluoride, 1,2-difluoroethylene, vinylidene fluoride, trifluoroethylene, pentafluoropropylene, and hexafluoroisobutylene;
- (per)fluoroalkylethylenes complying with formula CH₂=CH-R_{f0}, in which R_{f0} is a C₁-C₆ (per)fluoroalkyl or a C₁-C₆ (per)fluorooxyalkyl having one or more ether groups;
- chloro- and/or bromo- and/or iodo-C₂-C₆ fluoroolefins, like chlorotrifluoroethylene;
- fluoroalkylvinylethers complying with formula CF₂=CFOR_{f1}, in which R_{f1} is a C₁-C₆ fluoro- or perfluoroalkyl, e.g. -CF₃, -C₂F₅, -C₃F₇ ;
- hydrofluoroalkylvinylethers complying with formula CH₂=CFOR_{f1}, in which R_{f1} is a C₁-C₆ fluoro- or perfluoroalkyl, e.g. -CF₃, -C₂F₅, -C₃F₇ ;
- fluoro-oxyalkylvinylethers complying with formula CF₂=CFOX₀, in which X₀ is a C₁-C₁₂ oxyalkyl, or a C₁-C₁₂ (per)fluorooxyalkyl having one or more ether groups, like perfluoro-2-propoxy-propyl;
- fluoroalkyl-methoxy-vinylethers complying with formula CF₂=CFOCF₂OR_{f2} in which R_{f2} is a C₁-C₆ fluoro- or perfluoroalkyl, e.g. -CF₃, -C₂F₅, -C₃F₇ or a C₁-C₆ (per)fluorooxyalkyl having one or more ether groups, like -C₂F₅-O-CF₃;
- functional fluoro-alkylvinylethers complying with formula CF₂=CFOY₀, in which Y₀ is a C₁-C₁₂ alkyl or (per)fluoroalkyl, or a C₁-C₁₂ oxyalkyl or a C₁-C₁₂ (per)fluorooxyalkyl, said Y₀ group comprising a carboxylic or sulfonic acid group, in its acid, acid halide or salt form;
- fluorodioxoles, of formula: 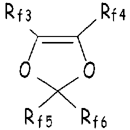 wherein each of R_{f3}, R_{f4}, R_{f5}, R_{f6}, equal or different each other, is independently a fluorine atom, a C₁-C₆ fluoro- or per(halo)fluoroalkyl, optionally comprising one or more oxygen atom, e.g. -CF₃, -C₂F₅, -C₃F₇, - OCF₃, -OCF₂CF₂OCF₃.

7. Polymer (P) according to any one of claims 5 or 6, which is a copolymer comprising recurring units deriving from compound (AX), recurring units deriving from at least one C₂-C₈ perfluoroolefin, preferably tetrafluoroethylene, and recurring units deriving from at least one functional fluoro-alkylvinylether of formula CF₂=CFOY₀ selected from the group consisting of:
(j) sulfonyl halide fluorovinylethers of formula: CF₂=CF-O-(CF₂)_{m'}SO₂X', wherein m' is an integer between 1 and 10, preferably between 1 and 6, more preferably between 2 and 4, even more preferably m equals 2; and X' is chosen among halogens (Cl, F, Br, I), preferably F, -O⁻M'⁺, wherein M'⁺ is a cation selected among H⁺, NH₄⁺, K⁺, Li⁺, Na⁺, or mixtures thereof, preferably M'⁺ is H⁺; and
(jj) sulfonyl fluoride fluoroalkoxyvinylethers of formula: CF₂=CF-(OCF₂CF(R_{F1}))_{w}-O-CF₂(CF(R_{F2}))_{y}SO₂X'
X' is chosen among halogens (Cl, F, Br, I), preferably F, -O⁻M'⁺, wherein M'⁺ is a cation selected among H⁺, NH₄⁺, K⁺, Li⁺, Na⁺, or mixtures thereof, preferably M'⁺ is H⁺; and wherein w is an integer between 0 and 2, R_{F1} and R_{F2}, equal or different from each other, are independently F, Cl or a C₁-C₁₀ fluoroalkyl group, optionally substituted with one or more ether oxygens, y is an integer between 0 and 6; preferably w is 1, R_{F1} is -CF₃, y is 1 and R_{F2} is F.

8. Polymer (P) according to any one of claims 5 to 7 which comprises:
- 10 to 20 % by moles of recurring units deriving from compound (AX),
- 60 to 70 % by moles of recurring units deriving from tetrafluoroethylene, and
- 15 to 25 % by moles of recurring units deriving from the functional fluoro-alkylvinylether of formula (FM1-D): 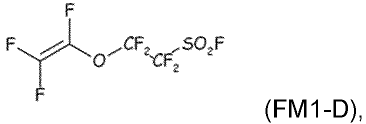 the amount by moles being referred to the total moles of recurring units of polymer (P).

9. A process for manufacturing polymer (P) of any one of claims 5 to 8, said process comprising the polymerization of a monomer mixture (MM) comprising:
(i) at least one compound (AX) according to claim 1;
(ii) at least one fluoromonomer (FM);
in the presence of at least one free radical initiator and optionally a surfactant.

10. A process for the chemical modification of polymer (P) as defined in any one of claims 5 to 8 comprising the hydrolysis of the xanthate moiety of the recurring units deriving from compound (AX), namely the moiety of formula 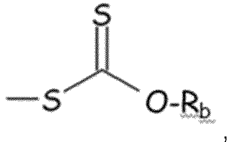 to the group -SH.

11. The process of claim 10 wherein the hydrolysis is performed by reacting polymer (P) with an acid or a base in an aqueous solution.

12. A process for the chemical modification of polymer (P) as defined in any one of claims 5 to 8 comprising the oxidation, preferably performed by reacting polymer (P) with hydrogen peroxide, of the xanthate moiety of the recurring units deriving from compound (AX), namely the moiety of formula 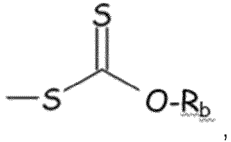 to the group -SO₃H.

13. A polymer (P^{ox}) of formula (VII): 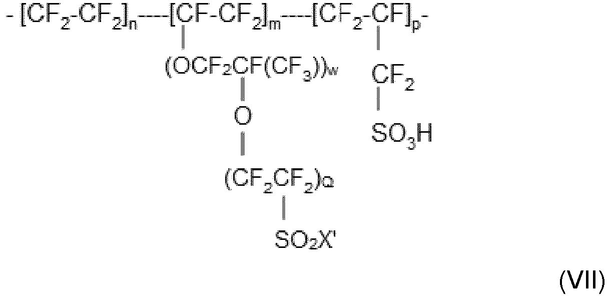 Wherein: w is either 0 or 1, Q is an integer between 1 and 4, preferably 1 or 2; X' is selected among halogens, preferably F, or is -O⁻M'⁺, wherein M'⁺ is a cation selected among H⁺, NH₄⁺, K⁺, Li⁺, Na⁺, or mixtures thereof, preferably M'⁺ is H⁺; n and p are, independently from each other, integers greater than 0; m is equal to greater than 0; m, n and p denoting the molar fraction of each monomer in the polymer.

14. An article, preferably a membrane for use in an electrolysis cell or a fuel cell, comprising polymer (P) of any one of claims 5 to 8 or polymer (P^{ox}) of claim 13.

15. Use of a compound (AX) of formula (I): 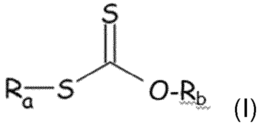 wherein Rₐ is a (per)fluorinated allyl group and R_{b} is a straight or branched alkyl group as chain transfer agent in controlled radical polymerizations.

## Patentansprüche

1. Verbindung (AX) der Formel (I): 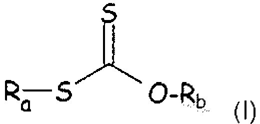 wobei Rₐ für eine (per)fluorierte Allylgruppe steht und R_{b} für eine gerade oder verzweigte Alkylgruppe steht, vorzugsweise R_{b} für eine gerade oder verzweigte C₁-C₁₂-Alkylgruppe steht.

2. Verbindung (AX) nach Anspruch 1, die der Formel (II) entspricht: 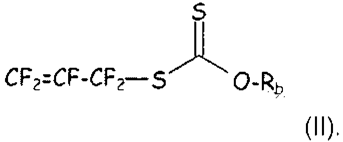 wobei R_{b} ausgewählt ist aus der Gruppe bestehend aus: Ethyl-, Isopropyl-, n-Butyl-, Isobutyl-, n-Pentyl- und Isopentylgruppen, vorzugsweise Ethyl.

3. Verfahren zur Herstellung der Verbindung (AX) nach Anspruch 1, wobei das Verfahren die folgenden Schritte a) und b) umfasst:
a) Bereitstellen eines Xanthatsalzes der Formel (IV) 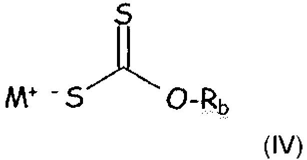 wobei R_{b} für eine gerade oder verzweigte Alkylgruppe steht und M⁺ für ein einwertiges Kation steht, das vorzugsweise aus Alkalimetallkationen ausgewählt ist, weiter bevorzugt M⁺ aus Na⁺, K⁺, Cs⁺ und Li⁺ ausgewählt ist, noch weiter bevorzugt M⁺ für K⁺ steht;
b) Umsetzen des in Schritt a) bereitgestellten Xanthatsalzes mit einem (Per)fluorallylfluorsulfat der Formel (V)
Rₐ-OSO₂X (V)
wobei Rₐ für eine (per)fluorierte Allylgruppe steht und X für ein Halogenatom steht.

4. Polymer (P), umfassend Wiederholungseinheiten, die sich von Verbindung (AX) der Formel (I) ableiten 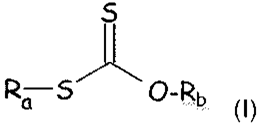 wobei Rₐ für eine (per)fluorierte Allylgruppe steht und R_{b} für eine gerade oder verzweigte Alkylgruppe steht.

5. Polymer (P) nach Anspruch 4, bei dem es sich um ein Copolymer handelt, das Wiederholungseinheiten, die sich von Verbindung (AX) ableiten, vorzugsweise in einer Menge von 85 bis 5 Mol-%, bezogen auf die gesamten Mole von Wiederholungseinheiten von Polymer (P), und Wiederholungseinheiten, die sich von mindestens einem ethylenisch ungesättigten Monomer mit mindestens einem Fluoratom [Fluormonomer (FM)] ableiten, vorzugsweise in einer Menge von 15 bis 95 Mol-%, bezogen auf die gesamten Mole von Wiederholungseinheiten von Polymer (P), umfasst.

6. Polymer (P) nach Anspruch 5, wobei das Fluormonomer (FM) ausgewählt ist aus der Gruppe bestehend aus:
- C₂-C₈-Perfluorolefinen, wie Tetrafluorethylen, Hexafluorpropen;
- wasserstoffhaltigen C₂-C₈-Fluorolefinen, wie Vinylfluorid, 1,2-Difluorethylen, Vinylidenfluorid, Trifluorethylen, Pentafluorpropylen und Hexafluorisobutylen;
- (Per) fluoralkylethylenen der Formel CH₂=CH-R_{f0}, worin R_{f0} für ein C₁-C₆-(Per)fluoralkyl oder ein C₁-C₆-(Per)fluoroxyalkyl mit einer oder mehreren Ethergruppe(n) steht;
- Chlor- und/oder Brom- und/oder iod-C₂-C₆-fluorolefinen, wie Chlortrifluorethylen;
- Fluoralkylvinylethern der Formel CF₂=CFOR_{f1}, worin R_{f1} für ein C₁-C₆-Fluor- oder -Perfluoralkyl, z. B. -CF₃, -C₂F₅, -C₃F₇, steht;
- Hydrofluoralkylvinylethern der Formel CH₂=CFOR_{f1}, worin R_{f1} für ein C₁-C₆-Fluor- oder -Perfluoralkyl, z. B. -CF₃, -C₂F₅, -C₃F₇, steht;
- Fluoroxyalkylvinylethern der Formel CF₂=CFOX₀, worin X₀ für ein C₁-C₁₂-Oxyalkyl oder ein C₁-C₁₂-(Per)fluoroxyalkyl mit einer oder mehreren Ethergruppe(n), wie Perfluor-2-propoxypropyl, steht;
- Fluoralkylmethoxyvinylethern der Formel CF₂=CFOCF₂OR_{f2}, worin R_{f2} für ein C₁-C₆-Fluor- oder - Perfluoralkyl, z. B. -CF₃, -C₂F₅, -C₃F₇, oder ein C₁-C₆-(Per)fluoroxyalkyl mit einer oder mehreren Ethergruppe(n), wie -C₂F₅-O-CF₃, steht;
- funktionellen Fluoralkylvinylethern der Formel CF₂=CFOY₀, worin Y₀ für ein C₁-C₁₂-Alkyl oder - (Per) fluoralkyl oder ein C₁-C₁₂-Oxyalkyl oder ein C₁-C₁₂-(Per)fluoroxyalkyl steht, wobei die Y₀-Gruppe eine Carbonsäure- oder Sulfonsäuregruppe in ihrer Säure-, Säurehalogenid- oder Salzform umfasst;
- Fluordioxolen der Formel: 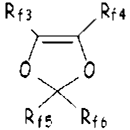 worin R_{f3}, R_{f4}, R_{f5} und R_{f6}, die gleich oder voneinander verschieden sind, jeweils unabhängig für ein Fluoratom oder ein C₁-C₆-Fluor- oder Per(halogen)fluoralkyl, das gegebenenfalls ein oder mehrere Sauerstoffatom(e) umfasst, z. B. -CF₃, -C₂F₅, -C₃F₇, -OCF₃, -OCF₂CF₂OCF₃, stehen.

7. Polymer (P) nach einem der Ansprüche 5 oder 6, bei dem es sich um ein Copolymer handelt, das umfasst: Wiederholungseinheiten, die sich von Verbindung (AX) ableiten, Wiederholungseinheiten, die sich von mindestens einem C₂-C₈-Perfluorolefin, vorzugsweise Tetrafluorethylen, ableiten, und Wiederholungseinheiten, die sich von mindestens einem funktionellen Fluoralkylvinylether der Formel CF₂=CFOY₀ ableiten, der ausgewählt ist aus der Gruppe bestehend aus:
(j) Sulfonylhalogenidfluorvinylethern der Formel: CF₂=CF-O- (CF₂)_{m'} SO₂X', worin m' für eine ganze Zahl zwischen 1 und 10, vorzugsweise zwischen 1 und 6, weiter bevorzugt zwischen 2 und 4, steht und noch weiter bevorzugt m gleich 2 ist und X' ausgewählt ist aus Halogenen (Cl, F, Br, I), vorzugsweise F, -O⁻M'⁺, wobei M'⁺ für ein Kation steht, das aus H⁺, NH₄⁺, K⁺, Li⁺, Na⁺ oder Mischungen davon ausgewählt ist, wobei vorzugsweise M'⁺ für H⁺ steht; und
(jj) Sulfonylfluoridfluoralkoxyvinylethern der Formel: CF₂=CF-(OCF₂CF(R_{F1}))_{w}-O-CF₂(CF(R_{F2}))_{y}SO₂X'
wobei X' ausgewählt ist aus Halogenen (Cl, F, Br, I), vorzugsweise F, -O⁻M'⁺, wobei M'⁺ für ein Kation steht, das aus H⁺, NH₄⁺, K⁺, Li⁺, Na⁺ oder Mischungen davon ausgewählt ist, wobei vorzugsweise M'⁺ für H⁺ steht und wobei w für eine ganze Zahl zwischen 0 und 2 steht, R_{F1} und R_{F2}, die gleich oder voneinander verschieden sind, unabhängig für F, Cl oder eine C₁-C₁₀-Fluoralkylgruppe, die gegebenenfalls durch ein oder mehrere Ether-Sauerstoffatom(e) substituiert ist, stehen, y für eine ganze Zahl zwischen 0 und 6 steht; vorzugsweise w für 1 steht, R_{F1} für -CF₃ steht, y für 1 steht und R_{F2} für F steht.

8. Polymer (P) nach einem der Ansprüche 5 bis 7, das umfasst:
- 10 bis 20 Mol-% Wiederholungseinheiten, die sich von Verbindung (AX) ableiten,
- 60 bis 70 Mol-% Wiederholungseinheiten, die sich von Tetrafluorethylen ableiten, und
- 15 bis 25 Mol-% Wiederholungseinheiten, die sich vom funktionellen Fluoralkylvinylether der Formel (FM1-D) ableiten: 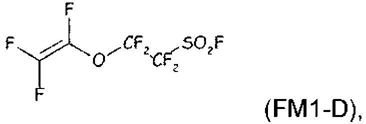 wobei sich die molare Menge auf die gesamten Mole von Wiederholungseinheiten von Polymer (P) bezieht.

9. Verfahren zur Herstellung von Polymer (P) nach einem der Ansprüche 5 bis 8, wobei das Verfahren die Polymerisation einer Monomermischung (MM) umfasst, umfassend:
(i) mindestens eine Verbindung (AX) nach Anspruch 1;
(ii) mindestens ein Fluormonomer (FM);
in Gegenwart mindestens eines Radikalinitiators und gegebenenfalls eines Tensids.

10. Verfahren zur chemischen Modifizierung von Polymer (P) nach einem der Ansprüche 5 bis 8, umfassend die Hydrolyse der Xanthatgruppierung der Wiederholungseinheiten, die sich von Verbindung (AX) ableiten, nämlich der Gruppierung der Formel 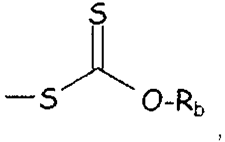 zu der Gruppe -SH.

11. Verfahren nach Anspruch 10, wobei die Hydrolyse durch Umsetzen von Polymer (P) mit einer Säure oder einer Base in einer wässrigen Lösung durchgeführt wird.

12. Verfahren zur chemischen Modifizierung von Polymer (P) nach einem der Ansprüche 5 bis 8, umfassend die Oxidation, vorzugsweise durch Umsetzen von Polymer (P) mit Wasserstoffperoxid durchgeführt, der Xanthatgruppierung der Wiederholungseinheiten, die sich von Verbindung (AX) ableiten, nämlich der Gruppierung der Formel 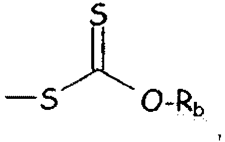 zu der Gruppe -SO₃H.

13. Polymer (P^{ox}) der Formel (VII): 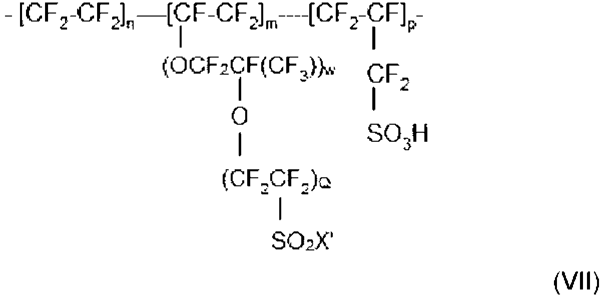 wobei: w entweder für 0 oder für 1 steht, Q für eine ganze Zahl zwischen 1 und 4, vorzugsweise 1 oder 2, steht; X' ausgewählt ist aus Halogenen, vorzugsweise F, oder für -O⁻M'⁺ steht, wobei M'⁺ für ein Kation steht, das aus H⁺, NH₄⁺, K⁺, Li⁺, Na⁺ oder Mischungen davon ausgewählt ist, wobei vorzugsweise M'⁺ für H⁺ steht; n und p unabhängig voneinander für ganze Zahlen größer 0 stehen; m gleich oder größer als 0 ist; wobei m, n und p den molaren Anteil jedes Monomers im Polymer bedeuten.

14. Gegenstand, vorzugsweise eine Membran zur Verwendung in einer Elektrolysezelle oder einer Brennstoffzelle, umfassend Polymer (P) nach einem der Ansprüche 5 bis 8 oder Polymer (P^{ox}) nach Anspruch 13.

15. Verwendung einer Verbindung (AX) der Formel (I): 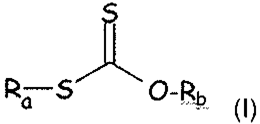 wobei Rₐ für eine (per)fluorierte Allylgruppe steht und R_{b} für eine gerade oder verzweigte Alkylgruppe steht, als Kettenübertragungsmittel bei kontrollierten Radikalpolymerisationen.

## Revendications

1. Composé (AX) de formule (I) : 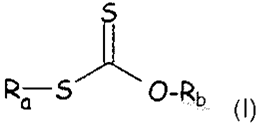 dans laquelle Rₐ est un groupe allyle (per) fluoré et R_{b} est un groupe alkyle linéaire ou ramifié, préférablement R_{b} est un groupe alkyle linéaire ou ramifié en C₁-C₁₂.

2. Composé (AX) selon la revendication 1 répondant à la formule (II) : 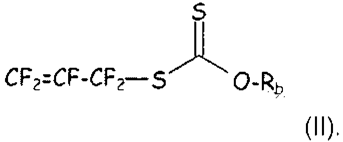 dans laquelle R_{b} est choisi dans le groupe constitué : des groupes éthyle, isopropyle, n-butyle, isobutyle, n-pentyle et isopentyle, de préférence éthyle.

3. Procédé de préparation du composé (AX) selon la revendication 1, ledit procédé comprenant les étapes a) et b) suivantes :
a) fourniture d'un sel de xanthate de formule (IV) 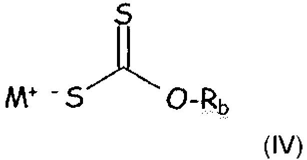 dans laquelle R_{b} est un groupe alkyle linéaire ou ramifié et M⁺ est un cation monovalent choisi de préférence parmi les cations de métaux alcalins, plus préférablement M⁺ est choisi parmi Na⁺, K⁺, Cs⁺ et Li⁺, encore plus préférablement M⁺ est K⁺ ;
b) mise en réaction du sel de xanthate fourni à l'étape a) avec un fluorosulfate de (per)fluoroallyle de formule (V)
Rₐ-OSO₂X (V)
dans laquelle Rₐ est un groupe allyle (per)fluoré et X est un atome d'halogène.

4. Polymère (P) comprenant des motifs répétitifs dérivés d'un composé (AX) de formule (I) 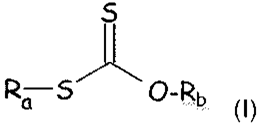 dans laquelle Rₐ est un groupe allyle (per)fluoré et R_{b} est un groupe alkyle linéaire ou ramifié.

5. Polymère (P) selon la revendication 4, qui est un copolymère comprenant des motifs répétitifs dérivés d'un composé (AX), de préférence en une quantité de 85 à 5 % en moles par rapport au nombre total de moles de motifs répétitifs du polymère (P), et des motifs répétitifs dérivés d'au moins un monomère éthyléniquement insaturé comprenant au moins un atome de fluor [fluoromonomère (FM)], de préférence en une quantité de 15 à 95 % en moles par rapport au nombre total de moles de motifs répétitifs du polymère (P).

6. Polymère (P) selon la revendication 5, dans lequel le fluoromonomère (FM) est choisi dans le groupe constitué de :
- des perfluorooléfines en C₂-C₈ telles que le tétrafluoroéthylène, l'hexafluoropropène ;
- des fluorooléfines en C₂-C₈ contenant de l'hydrogène telles que le fluorure de vinyle, le 1,2-difluoroéthylène, le fluorure de vinylidène, le trifluoroéthylène, le pentafluoropropylène et l'hexafluoroisobutylène ;
- des (per)fluoroalkyléthylènes répondant à la formule CH₂=CH-R_{f0}, dans laquelle R_{f0} est un (per)fluoroalkyle en C₁-C₆ ou un (per)fluorooxyalkyle en C₁-C₆ ayant un ou plusieurs groupes éther ;
- des chloro- et/ou bromo- et/ou iodo-C₂-C₆ fluorooléfines, comme le chlorotrifluoroéthylène ;
- des fluoroalkylvinyléthers répondant à la formule CF₂=CFOR_{f1}, dans laquelle R_{f1} est un fluoroalkyle ou perfluoroalkyle en C₁-C₆, par exemple -CF₃, -C₂F₅, -C₃F₇ ;
- des hydrofluoroalkylvinyléthers répondant à la formule CH₂=CFOR_{f1}, dans laquelle R_{f1} est un fluoroalkyle ou perfluoroalkyle en C₁-C₆, par exemple -CF₃, -C₂F₅, -C₃F₇ ;
- des fluoro-oxyalkylvinyléthers répondant à la formule CF₂=CFOX₀, dans laquelle X₀ est un oxyalkyle en C₁-C₁₂ ou un (per) fluorooxyalkyle en C₁-C₁₂ ayant un ou plusieurs groupes éthers, comme perfluoro-2-propoxypropyle ;
- des fluoroalkyl-méthoxy-vinyléthers répondant à la formule CF₂=CFOCF₂OR_{f2} dans laquelle R_{f2} est un fluoroalkyle ou perfluoroalkyle en C₁-C₆, par exemple - CF₃, -C₂F₅, -C₃F₇ ou un (per)fluorooxyalkyle en C₁-C₆ ayant un ou plusieurs groupes éthers, comme -C₂F₅-O-CF₃;
- des fluoro-alkylvinyléthers fonctionnalisés répondant à la formule CF₂=CFOY₀, dans laquelle Y₀ est un alkyle ou un (per)fluoroalkyle en C₁-C₁₂ ou un oxyalkyle en C₁-C₁₂ ou un (per)fluorooxyalkyle en C₁-C₁₂, ledit groupe Y₀ comprenant un groupe acide carboxylique ou sulfonique, sous sa forme d'acide, d'halogénure d'acide ou de sel ;
- des fluorodioxoles, de formule : 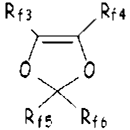 chacun parmi R_{f3}, R_{f4}, R_{f5} et R_{f6}, égaux ou différents les uns des autres, étant indépendamment un atome de fluor, un fluoro- ou per(halogéno)fluoroalkyle en C₁-C₆, éventuellement comprenant un ou plusieurs atomes d'oxygène, par exemple -CF₃, -C₂F₅, -C₃F₇, -OCF₃, - OCF₂CF₂OCF₃.

7. Polymère (P) selon l'une quelconque des revendications 5 ou 6, qui est un copolymère comprenant des motifs répétitifs dérivés d'un composé (AX), des motifs répétitifs dérivés d'au moins une perfluorooléfine en C₂-C₈, de préférence le tétrafluoroéthylène, et des motifs répétitifs dérivés d'au moins un fluoro-alkylvinyléther fonctionnel de formule CF₂=CFOY₀ choisi dans le groupe constitué :
(j) des halogénures de fluorovinyléthersulfonyle de formule : CF₂=CF-O-(CF₂)_{m'} SO₂X', m' étant un entier compris entre 1 et 10, préférablement entre 1 et 6, plus préférablement entre 2 et 4, encore plus préférablement m étant égal à 2 ; et X' étant choisi parmi les halogènes (Cl, F, Br, I), préférablement F, -O⁻M'⁺, M'⁺ étant un cation choisi parmi H⁺, NH₄⁺, K⁺, Li⁺, Na⁺ ou des mélanges de ceux-ci, de préférence M'⁺ étant H⁺ ; et
(jj) des fluorures de fluoroalcoxyvinyléthersulfonyle de formule : CF₂=CF-(OCF₂CF(R_{F1}))_{w}-O-CF₂(CF(R_{F2}))_{y}SO₂X'
X' est choisi parmi les halogènes (Cl, F, Br, I), de préférence F,-O⁻M'⁺, M'⁺ étant un cation choisi parmi H⁺, NH₄⁺, K⁺, Li⁺, Na⁺ ou des mélanges de ceux-ci, de préférence M'⁺ étant H⁺ ; et w étant un entier entre 0 et 2, R_{F1} et R_{F2}, égaux ou différents l'un de l'autre, étant indépendamment F, Cl ou un groupe fluoroalkyle en C₁-C₁₀, éventuellement substitué par un ou plusieurs oxygènes d'éther, y étant un entier compris entre 0 et 6 ; préférablement w étant 1, R_{F1} étant -CF₃, y étant 1 et R_{F2} étant F.

8. Polymère (P) selon l'une quelconque des revendications 5 à 7, qui comprend :
- 10 à 20 % en moles de motifs répétitifs dérivés d'un composé (AX),
- 60 à 70 % en moles de motifs répétitifs dérivés du tétrafluoroéthylène, et
- 15 à 25 % en moles de motifs répétitifs dérivés du fluoro-alkylvinyléther fonctionnalisé de formule (FM1 - D) : 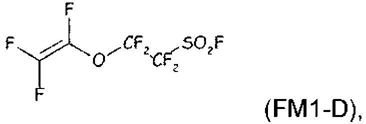 la quantité en moles étant rapportée au nombre total de moles de motifs répétitifs du polymère (P).

9. Procédé de fabrication de polymère (P) selon l'une quelconque des revendications 5 à 8, ledit procédé comprenant la polymérisation d'un mélange de monomères (MM) comprenant :
(i) au moins un composé (AX) selon la revendication 1 ;
(ii) au moins un fluoromonomère (FM) ;
en présence d'au moins un initiateur de radicaux libres et éventuellement d'un tensioactif.

10. Procédé pour la modification chimique du polymère (P) tel que défini dans l'une quelconque des revendications 5 à 8, comprenant l'hydrolyse du groupement xanthate des motifs répétitifs dérivés d'un composé (AX), à savoir le groupement de formule 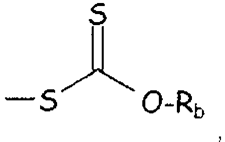 en le groupe -SH.

11. Procédé selon la revendication 10, dans lequel l'hydrolyse est effectuée en mettant en réaction le polymère (P) avec un acide ou une base dans une solution aqueuse.

12. Procédé de modification chimique du polymère (P) tel que défini dans l'une quelconque des revendications 5 à 8 comprenant l'oxydation, de préférence effectuée par mise en réaction du polymère (P) avec du peroxyde d'hydrogène, du groupement xanthate des motifs répétitifs dérivés du composé (AX), à savoir le groupement de formule 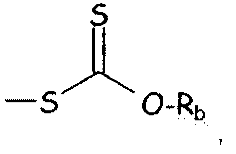 en le groupe -SO₃H.

13. Polymère (P^{ox}) de formule (VII) : 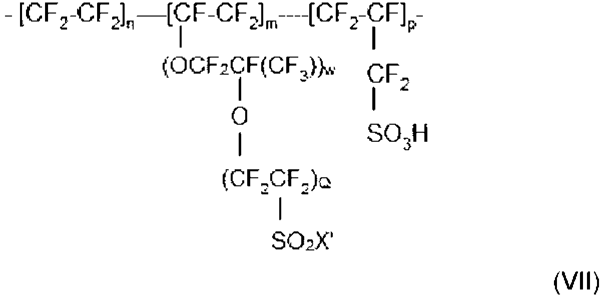 dans laquelle : w est soit 0, soit 1, Q est un entier compris entre 1 et 4, de préférence 1 ou 2 ; X' est choisi parmi des halogènes, préférablement F, ou est -O⁻M'⁺, M'⁺ étant un cation choisi parmi H⁺, NH₄⁺, K⁺, Li⁺, Na⁺ ou des mélanges de ceux-ci, préférablement M'⁺ étant H⁺ ; n et p étant, indépendamment l'un de l'autre, des entiers supérieurs à 0 ; m étant égal à supérieur à 0 ; m, n et p désignant la fraction molaire de chaque monomère dans le polymère.

14. Article, de préférence membrane pour utilisation dans une cellule d'électrolyse ou une pile à combustible, comprenant un polymère (P) selon l'une quelconque des revendications 5 à 8 ou un polymère (P^{ox}) selon la revendication 13.

15. Utilisation d'un composé (AX) de formule (I) : 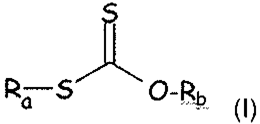 dans laquelle Rₐ est un groupe allyle (per)fluoré et R_{b} est un groupe alkyle linéaire ou ramifié en tant qu'agent de transfert de chaîne dans des polymérisations radicalaires contrôlées.
